(19) Europäisches Patentamt
European Patent Office
Office européen des brevets

(11) **EP 4 346 604 B1**

(12) **EUROPEAN PATENT SPECIFICATION**

(45) Date of publication and mention
of the grant of the patent:
**19.11.2025 Bulletin 2025/47**

(21) Application number: **22736347.0**

(22) Date of filing: **29.06.2022**

(51) International Patent Classification (IPC):
*A61B 5/372* (2021.01)    *A61B 5/00* (2006.01)
*G06N 3/0464* (2023.01)    *G06N 3/084* (2023.01)
*G16H 50/20* (2018.01)    *G16H 50/70* (2018.01)
*G16H 20/70* (2018.01)

(52) Cooperative Patent Classification (CPC):
**A61B 5/372; A61B 5/725; A61B 5/7267;
G06N 3/0464; G06N 3/084; G16H 20/70;
G16H 50/20; G16H 50/70**

(86) International application number:
**PCT/GB2022/051672**

(87) International publication number:
**WO 2023/007118 (02.02.2023 Gazette 2023/05)**

(54) **LEARNABLE FILTERS FOR EEG CLASSIFICATION**

LERNFÄHIGE FILTER FÜR EEG-KLASSIFIZIERUNG

FILTRES POUVANT ÊTRE APPRIS DESTINÉS À LA CLASSIFICATION D'EEG

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.07.2021 GR 20210100506
21.09.2021 GB 202113420**

(43) Date of publication of application:
**10.04.2024 Bulletin 2024/15**

(73) Proprietor: **Cogitat LTD
Market Hill Royston SG8 9JN (GB)**

(72) Inventors:
• **ADAMOS, Dimitrios**
**Royston, SG8 9JN (GB)**
• **LASKARIS, Nikolaos**
**Royston, SG8 9JN (GB)**
• **ZAFEIRIOU, Stefanos**
**Royston, SG8 9JN (GB)**
• **LUDWIG, Siegfried**
**Royston, SG8 9JN (GB)**
• **BAKAS, Stylianos**
**Royston, SG8 9JN (GB)**

(74) Representative: **Varley, James Richard et al
Venner Shipley LLP
200 Aldersgate
London EC1A 4HD (GB)**

(56) References cited:
• **BORRA DAVIDE ET AL: "Interpretable and
lightweight convolutional neural network for
EEG decoding: Application to movement
execution and imagination", NEURAL
NETWORKS, ELSEVIER SCIENCE PUBLISHERS,
BARKING, GB, vol. 129, 29 May 2020
(2020-05-29), pages 55 - 74, XP086239502, ISSN:
0893-6080, [retrieved on 20200529], DOI: 10.1016/
J.NEUNET.2020.05.032**
• **ROBIN TIBOR SCHIRRMEISTER ET AL: "Deep
learning with convolutional neural networks for
EEG decoding and visualization : Convolutional
Neural Networks in EEG Analysis", HUMAN
BRAIN MAPPING, vol. 38, no. 11, 7 August 2017
(2017-08-07), pages 5391 - 5420, XP055537466,
ISSN: 1065-9471, DOI: 10.1002/hbm.23730**

- **VERNON J LAWHERN ET AL: "EEGNet: a compact convolutional neural network for EEG-based brain-computer interfaces", JOURNAL OF NEURAL ENGINEERING, INSTITUTE OF PHYSICS PUBLISHING, BRISTOL, GB, vol. 15, no. 5, 27 July 2018 (2018-07-27), pages 56013, XP020330961, ISSN: 1741-2552, [retrieved on 20180727], DOI: 10.1088/1741-2552/AACE8C**

- **LOTTE F ET AL: "A review of classification algorithms for EEG-based brain-computer interfaces: a 10 year update", JOURNAL OF NEURAL ENGINEERING, INSTITUTE OF PHYSICS PUBLISHING, BRISTOL, GB, vol. 15, no. 3, 16 April 2018 (2018-04-16), pages 31005, XP020327399, ISSN: 1741-2552, [retrieved on 20180416], DOI: 10.1088/1741-2552/AAB2F2**

**Description**

**Field**

This specification relates to the classification and/or decoding of brain activity signals, in particular electroencephalogram (EEG) signals, using machine-learning techniques.

**Background**

Patterns of brain activity are associated with different brain processes and can be used to identify different brain states and make behavioural predictions. However, the relevant features are not readily apparent and accessible. Conventional machine learning approaches to classifying brain activity involve extensive manual feature engineering, which may miss important as-yet unidentified features in captured brain activity signals, while deep learning approaches require large amounts of data and provide limited interpretability.

"Interpretable and lightweight convolutional neural network for EEG decoding: Application to movement execution and imagination" (D. Borra et al., Neural Netw. 2020 Sep;129:55-74) discloses a lightweight and interpretable shallow CNN (Sinc-ShallowNet), by stacking a temporal sine-convolutional layer (designed to learn band-pass filters, each having only the two cut-off frequencies as trainable parameters), a spatial depthwise convolutional layer (reducing channel connectivity and learning spatial filters tied to each band-pass filter), and a fully-connected layer finalizing the classification.

"Deep Learning with Convolutional Neural Networks for EEG Decoding and Visulaization" (R. T. Schirrmeister et al., Hum. Brain Mapp., 38: 5391-5420.) discloses deep ConvNets with a range of different architectures, designed for decoding imagined or executed tasks from raw EEG.

**Summary**

An end-to-end trainable model with strong constraints that can be trained on limited data and provides full insight into relevant features is provided. The invention is defined in the appended claims.

According to one aspect of this specification, there is described a computer implemented method of classifying brain activity signals. The method comprises: receiving a plurality of channels of brain activity signals; generating a plurality of channels of filtered brain activity signals by applying a plurality of filters to the received channels of brain activity signals, wherein the plurality of filters comprises a plurality of trained parameterised bandpass filters; determining, using an untrained differentiable feature module, a plurality of feature maps from the plurality of channels of filtered brain activity signals; and determining, using a classification model, one or more classifications for the received plurality of channels of brain activity signals based on the determined feature maps. The brain activity signals may be EEG signals.

According to a further aspect of this specification, there is described a computer implemented method of training a model for classifying brain activity signals. The method comprises: for each of one or more of training examples from a training set, each training example comprising a plurality of channels of brain activity signals and a ground-truth classification: generating a plurality of channels of filtered brain activity signals by applying a plurality of filters to the plurality of brain activity signals of said training example, wherein the plurality of filters comprises a plurality of parametrised bandpass filters; determining, using an untrained differentiable feature module, a plurality of feature maps from the plurality of channels of filtered brain activity signals, wherein the differentiable feature module applies one or more fixed functions to the filtered brain activity signals to determine the feature maps; and determining, using a classification model, one or more candidate classifications for the plurality of channels of brain activity signals of said training example based on the determined feature maps. The method further comprises updating parameters of the plurality of filters and the classification model based on a comparison of the one or more candidate classifications to corresponding ground-truth classifications, wherein the comparison is made using an objective function and wherein the updates are determined using backpropagation of gradients. The brain activity signals may be EEG signals.

The objective function may comprise a comparison term comprising a norm of a difference between the classifications and corresponding ground-truth classifications.

The objective function may further comprise a regularisation term comprising a sum of norms of weights of the classification model. Other classification losses may alternatively be used, such as cross-entropy.

The foregoing and other implementations can each optionally include one or more of the following features, alone or in combination. Each of a plurality of feature maps in the plurality of feature maps may be associated with a respective plurality of channels of filtered brain activity signals channels corresponding to the plurality of channels of brain activity signals. Each of the respective plurality of channels of filtered brain activity signals channels may be determined by applying a respective plurality of filters to the plurality of channels of brain activity signals.

The one or more feature maps may comprise one or more measures of functional connectivity between brain activity signals in the plurality of filtered brain activity signals. Determining the one or more feature maps may comprise: determining a connectivity matrix between two or more of the filtered EEG signals; and extracting a feature vector from the connectivity matrix. The feature vector may correspond to an upper triangular or lower triangular of the connectivity matrix. Determining, using the classification model, the one or more classifications for the plurality of channels of brain activity signals may comprise inputting the extracted feature vectors into the classification model.

The one or more measures of functional connectivity between filtered brain activity signals may comprise one or more of: a correlation function between two of the filtered brain activity signals; a phase locking value; amplitude envelope correlations; and/or signal envelope correlations.

The one or more feature maps may comprise one or more of: a measure of magnitude of each of the filtered brain activity signals; a signal power; a signal variance; and/or a signal entropy. The measure of magnitude of each of the filtered brain activity signals may comprise a sum of the magnitude of the filtered brain activity signals signal over frequency bins of the filtered brain activity signal.

The plurality of channels of brain activity signals may be processed in the frequency domain, the time domain, or in a mix of the frequency and time domains.

One or more of the parameters of a filter may control a phase response of the filter. The phase response may be freely controlled. Examples of phase responses include zero-phase and linear-phase filters.

The one or more classifications for the plurality of channels of brain activity signals may comprise: a classification of a resting or active state; a classification of a dynamic state triggered by/underlying the physical or imaginary movement of extremities; a classification of a dynamic state triggered by/underlying a conscious or non-conscious cognitive process related to attention tasks, perception tasks, planning tasks, memory tasks, language tasks, arithmetic tasks, reading tasks, control interface tasks, and specialized tasks like flight or driving, either in a simulator or in a real vehicle action; a classification of an affective state; a classification of an anomaly; a classification of a control intention for an external device; and/or a classification of clinical states.

The plurality of filters may comprise a plurality of generalised Gaussian filters.

According to a further aspect of this specification, there is disclosed a computer implemented method of classifying signal data, the method comprising: receiving a plurality of channels of signal data; generating a plurality of channels of filtered signal data by applying a plurality of filters to the received channels of EEG signals, wherein the plurality of filters comprises a plurality of learned generalised Gaussian filters; determining, using a differentiable feature module, a plurality of feature maps from the plurality of channels of filtered signal data; and determining, using a classification model, one or more classifications for the received plurality of channels of signal data based on the determined feature maps.

According to further aspects of this specification, there are disclosed computer program products comprising computer-readable code that, when executed by a computing system, causes the computing system to perform one or more of the methods disclosed herein.

According to further aspects of this specification, there are disclosed systems comprising one or more processors and a memory, the memory storing computer readable instructions that, when executed by the one or more processors, causes the system to perform one or more of the methods disclosed herein.

The use of parametrised filters in this way can result in models with an improved brain activity signal classification performance when compared with prior methods, while keeping the number of parameters of the model low. They also provide clear interpretability of the trained features in terms of relevant frequencies and/or functional connectivity. Using differentiable implementations of neuroscientifically plausible EEG features (e.g. band magnitude and signal correlations), the methods disclosed herein can discover classification-relevant frequency bands and functional connectivity patterns among a large repertoire of possible features, offering clear insights into feature importance and interpretations.

Throughout this description, examples and embodiments are described in the context of EEG signals. However, other brain activity signals, such as magnetoencephalography (MEG) signals, may alternatively or additionally be used.

## Brief Description of the Drawings

Embodiments will now be described by way of non-limiting examples with reference to the accompanying drawings, in which:

FIG. 1 shows a schematic overview of a method for classifying/decoding brain activity signals;
FIG. 2 shows examples of parameterised filters;
FIG. 3 shows a schematic overview of a method for training a model for classifying/decoding brain activity signals;
FIG. 4 shows a flow diagram of an example method of classifying/decoding brain activity signals;
FIG. 5 shows a flow diagram of an example method of training a model for classifying brain activity signals; and
FIG. 6 shows a schematic overview of a system for performing any of the methods disclosed herein.

**Detailed Description**

Patterns of brain activity are traditionally associated with different brain processes and can be used to differentiate brain states and make behavioural predictions. However, the relevant features are not readily apparent and accessible from brain activity (e.g. EEG) recordings, which may simply record electric potential differences at multiple locations on the skull of a subject.

To mine useful frequency magnitude and coupling features from multichannel EEG recordings, for example for EEG classification, a differentiable EEG decoding/classification pipeline is disclosed herein. The EEG pipeline is structured similarly to deep neural network architectures. Parameterised filters are used, such as generalised Gaussian functions, which include Morlet wavelets and sinc filters as special cases, while offering a smooth derivative for improved model training. This allows for end-to-end learnable discovery of interpretable features that can be used for classification of EEG signals.

In particular, the use of generalised Gaussian filters in some embodiments offers a smooth derivative for stable end-to-end model training and allows for learning interpretable features. The use of generalised Gaussian filters can result in improved trainability, while maintaining comparable or better classification accuracy than current state of the art EEG classification models. In some embodiments, the use of generalised Gaussian filters can result in a model with fewer parameters than some current state of the art EEG classification models. The use of such generalised Gaussian filters can also provide similar improvements in other signal classification tasks outside the field of brain activity.

This end-to-end differentiable EEG decoding/ classification pipeline makes use of explicit neuroscientific measures of brain activity, such as signal correlations. This type of feature itself is untrainable, but by using a differentiable implementation, the frequencies over which the connectivity is computed can be learned. The proposed model consists of learnable temporal filters, a differentiable feature module and a classification layer. Frequency filtering and the feature module are separated to make the targeted features fully explicit. As a result, the approaches disclosed herein can easily incorporate multiple types of features (e.g. local activity, covariation, phase synchrony, etc.) and keep close relation with the topographical information about the sensors.

The approaches disclosed herein can be interpreted both from the perspectives of conventional neuroscience on the one hand and deep learning on the other. The steps that make up an EEG pipeline (e.g. signal filtering, connectivity measure, classification) are analogous to consecutive layers in a deep neural network. Conversely, viewed from a deep learning perspective, our proposal for a differentiable EEG pipeline can be seen as introducing strong inductive biases into an end-to-end trainable model.

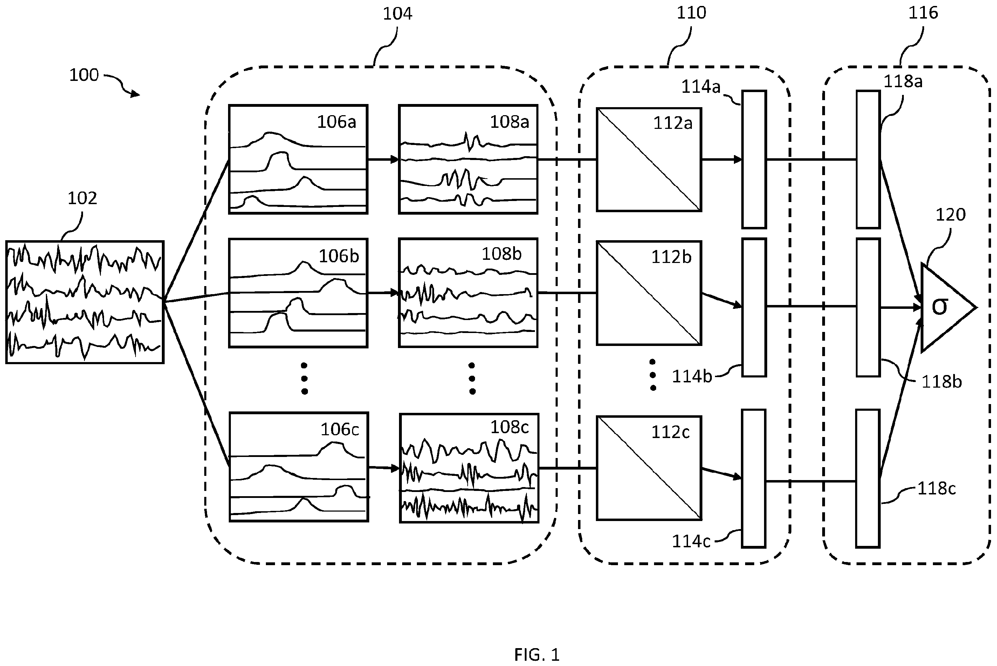

FIG. 1 shows a schematic overview of a method 100 for decoding/classifying EEG signals. A set of input EEG signals 102 (also referred to herein as "received EEG signals") is input into a filtering module 104, which applies a plurality of filters 106 to the input EEG signals 102 to generate a plurality of channels of filtered EEG signals 108. The filtered EEG signals are input into a differentiable feature module 110, which determines a plurality of feature maps 114 from the plurality of channels of filtered EEG signals 108 using a differentiable mapping 112. A classification model 116 determines one or more classifications of the input EEG signals 102 from the feature maps 114.

The EEG signals 102 comprise a plurality of channels of EEG data. Each channel is associated with a corresponding electrode/probe from which the EEG signals were captured. Each input plurality of channels of EEG signals 102 may correspond to a fixed-length time window, e.g. 20 seconds of captured EEG data. The EEG data may be supplied in substantially real time, e.g. streamed from electrodes attached to a subject as the electrodes capture the EEG signals.

Raw EEG signals may be pre-processed prior to use in the method. Multichannel signals may be filtered using a zero-phase band-pass filter (e.g. 3rd order Butterworth). To remove artefacts, independent component analysis (ICA) may be used.

The filtering module 104 may apply filters 106 in the frequency domain.

In some embodiments, such as the example shown in FIG. 1, multiple sets of parametrised bandpass filters 106a-c are applied to the input EEG signals 102. Each set of filters 106a-c comprises a plurality of parametrised bandpass filters, one for each channel of the input EEG signals 102. Each set of filters 106a-c is associated with a corresponding feature map 114a-c output by the feature module 110. Such feature maps may be referred to as "inter-channel" feature maps.

For example, a first set of filters 106a may be applied to the input EEG signals 102 to generate a first set of filtered EEG signals 108a. A differentiable mapping 112a is applied to the first set of filtered EEG signals 108a to generate a first feature map 114a. Similarly, a second feature map 114b is generated from the input EEG signals using a second set of filters 106b, and so on.

Alternatively or additionally, separate feature maps 114 may be generated for each channel of the input EEG signal 102 using corresponding filters 106 associated with each channel. Such feature maps may be referred to as "intra-channel" feature maps.

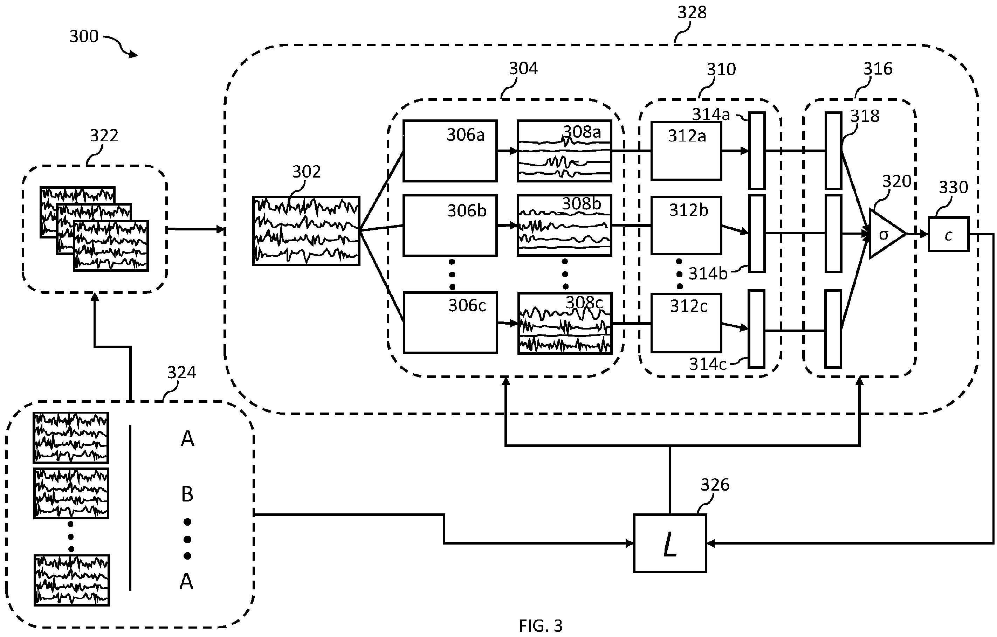

The plurality of filters 106 comprises a plurality of learned parameterised bandpass filters. The parameters of the filters 106 may have been learned using a machine learning method, such as the method described in relation to FIG. 3.

Examples of such filters that may be used include, but are not limited to convolutional filters, sinc bandpass filters, wavelets, and/or generalised Gaussians.

In some embodiments, the plurality of parametrised filters comprises a plurality of generalised Gaussian filters. A generalised Gaussian filter is a Gaussian-like filter in which the exponent of the exponent in the Gaussian (referred to herein as a "shape parameter") can take a range of values, and is not restricted to be two. An example of a generalised Gaussian, $G(x)$, is given by:

$$G(x) = \frac{\beta}{2\alpha\Gamma(1/\beta)} \, e^{-(|x-\mu|/\alpha)^\beta}$$

where $\Gamma(n) = (n\text{-}1)!$ is the gamma function, $\mu$ is a centre frequency, $\alpha$ is a scale parameter and $\beta$ is the shape parameter. In some embodiments, the gain of all the filters is fixed to one, and the normalisation factor may be dropped, giving the filter $F(x)$:

$$F(x) = \, e^{-(|x-\mu|/\alpha)^\beta}.$$

In some embodiments, the scale factor may be reparametrized in terms of a full-width at half maximum parameter, h, (also referred to herein as a "bandwidth parameter") using a generalisation of Cohen's re-parameterisation:

$$\alpha = \frac{h}{2 \ln 2^{1/\beta}}.$$

At $\beta = 2$ the generalised Gaussian resembles the normal distribution, which corresponds to a Morlet wavelet in the time domain. For higher values of the shape parameter, the filter moves towards a rectangular function, similar to a sinc filter in the frequency domain. In fact, the sinc filter is a special case of the generalized Gaussian in the limit of $\beta = \infty$.

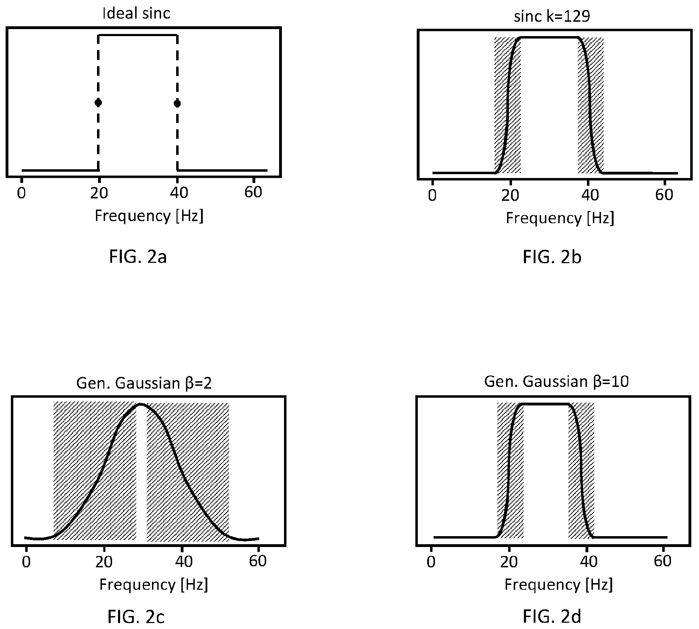

FIG. 2 shows a comparison of examples of generalised Gaussian filters to sinc filters. In all of the examples, the centre frequency is 30 Hz and the width of the filter is 20 Hz. The shaded areas denote regions of the filters with non-zero gradients/gradients that are not approximately zero.

FIG. 2a shows an example of idealised sinc bandpass filter. It has a value of zero everywhere except in the range 20-40 Hz, where it has a constant, non-zero value. The gradient of the sinc filter is thus zero everywhere, except at the transition frequencies 20 Hz and 40 Hz, where it is undefined. This makes idealised sinc filters difficult to train.

FIG. 2b shows an example of a truncated sinc bandpass filter. In this example, the filter has been truncated in the time domain to kernel size 129. The truncated sinc bandpass filter has a non-zero gradient in the transition regions around 20 Hz and 40 Hz, but is effectively zero elsewhere. This can make truncated sinc filters difficult to train, as the filter will get limited gradients during training.

FIG. 2c shows an example of a generalised Gaussian bandpass filter with $\beta = 2$. At this value of the shape parameter, the generalised Gaussian reduces to a standard Gaussian. The region over which the gradient of this generalised Gaussian is substantially non-zero is much greater than that of the idealised or truncated sinc bandpass filters, which can aid training of the parameters of the filter.

FIG. 2d shows an example of a generalised Gaussian bandpass filter with $\beta = 10$. At this value of the shape parameter, the generalised Gaussian has a similar form to the truncated sinc bandpass filter, with the region over which the gradient of this generalised Gaussian is substantially non-zero being similar to that of the truncated sinc filter.

The use of generalised Gaussian filters with a trainable shape parameter can move the filter from the Gaussian towards a rectangular shape over the course of training, reducing the cost of a wide transition band associated with its reduced frequency selectivity.

The plurality of parametrised filters may operate in the frequency domain. Designing the filters in the frequency domain allows for control over the phase response. Linear-phase filters, zero-phase or any linear or non-linear phase response may be used. For example, linear-phase filters here with a group delay of 20ms may be used to avoid distorting the signal. The group delay of a linear-phase setup may, in some embodiments, be used as a trainable parameter to allow for phase alignment between signals.

Returning to FIG. 1, the feature module 110 receives as input the filtered EEG signals 108 output by the filtering module 104, and uses them to construct feature maps 114a-c, each corresponding to a set of filtered EEG signals 112a-c. The feature module 110 applies a fixed differentiable mapping/function 112 to the filtered EEG signals 108 to generate the feature maps 114a-c. The use of a well-behaved differentiable mapping/function 112 allows gradients to backpropagate through the feature module 110 during training.

The fixed differentiable mapping/function 112 may comprise one or more measures of functional connectivity between EEG signals in the plurality of filtered EEG signals 108. The measure of functional connectivity may, in some embodiments, be represented as a connectivity matrix, as shown in FIG. 1. The upper (or lower) triangular of such a connectivity matrix may be extracted to be the feature map 114.

Examples of such functions include signal correlations, which may be computed as the Pearson correlation between two filtered signals in a set of filtered signals 108. In some embodiments, the correlation measure between two signals, $X$ and $Y$, may be given by:

$$r_{XY} = \frac{cov(X, Y)}{\sigma_X \sigma_y} = \frac{\sum_t (x_t - \bar{x})(y_t - \bar{y})}{\sqrt{\sum_t (x_t - \bar{x}) \sum_t (y_t - \bar{y})}}$$

over time $t$, where $\bar{x}$ and $\bar{y}$ are signal means. This measure can be efficiently computed using an inner product matrix multiplication for the numerator. Such signal correlations are defined in the range [-1,1], with larger absolute values indicating stringer coupling. In some embodiments, the absolute magnitude of $r_{XY}$ is taken ($|r_{XY}|$) to avoid negative values due to phase shifts between signals.

Alternatively or additionally, the one or more measures of functional connectivity may comprise a measure of phase relationships between signals in each set of filtered EEG signals 108. An example of such a measure is the phase locking value (PLV). The PLV between two signals is determined from the difference between the instantaneous phases of two signals, $\Delta\phi$. For example, the PLV may be given by:

$$PLV = \frac{1}{T} \left| \sum_t e^{i\Delta\phi} \right|$$

where $T$ is the length in time of the signals. Two signals with a strong coupling exhibit stable phase differences, leading to a high PLV, which is defined in the range [0, 1].

Other measures of functional connectivity may alternatively or additionally be used, including, but not limited to, amplitude envelope correlations and/or signal envelope correlations.

The fixed differentiable mapping/function 112 may alternatively or additionally comprise a measure of magnitude, a signal power, a signal variance, and/or a signal entropy of one or more (e.g. each) of the filtered EEG signals. In these examples, the feature maps may comprise a feature vector whose components are the measure of magnitude, the signal power, the signal variance, and/or the signal entropy of each of the channels of filtered EEG signals. In some embodiments, the measure of magnitude comprises a band-limited magnitude measure comprising a sum of the magnitude of a filtered EEG signal 108 over frequency bins of the filtered EEG signal 108. An example of such a measure, M, is given by:

$$M = \frac{1}{|\Omega|} \sum_\omega |x(\omega)|$$

where $\omega \in \Omega$ are the frequency bins and x is a filtered EEG signal in the frequency domain.

The feature maps 114 are input into a classification module 116, which uses them to determine one or more classifications for the received plurality of channels of EEG signals 102. The classifier is a parametrised model that provides an output indicative of which class of a plurality of classes the received EEG signals 102 belongs to. For example, the output may be a distribution over a plurality of classes, indicating a probability of the received EEG signals 102 belonging to that class. The parameters of the classifier may also be referred to herein as weights.

In some embodiments, the output classification is an indication of an intended action for an external device, e.g. a classification of a control intention for an external device. This classification may be converted into control signals for controlling the external device to perform the intended action. Examples of such external devices include, but are not limited to, external computing devices, vehicles (either simulated or real) and/or artificial limbs.

In some embodiments, the output classification is a classification of an anomaly in the EEG signals. Such anomalies may, for example, include seizures.

In some embodiments, the output classification is a classification of a clinical state and/or a diagnostic classification. Such clinical states/diagnoses may include, for example, Attention deficit hyperactivity disorder, dementia, sleep disorders, Autism Spectrum Disorder or the like.

Other examples of potential classifications include, but are not limited to a classification of a resting or active state; a classification of a dynamic state triggered by/underlying the physical or imaginary movement of extremities; a classification of a dynamic state triggered by/underlying a conscious or non-conscious cognitive process related to attention tasks, perception tasks, planning tasks, memory tasks, language tasks, arithmetic tasks, reading tasks, control interface tasks, and specialized tasks like flight or driving, either in a simulator or in a real vehicle action; and/or a classification of an affective state.

In the example shown, the classification module 116 comprises a linear layer 118 with sigmoid activation 120. This classifier essentially performs logistic regression, and has the advantage that the trained classifier weights can directly be used as importance weights attributed to the features. However, different features can have different distributions, they can be standardized in order to allow for the usage of the regression coefficients directly as an importance measure. The standardization may be performed after the feature module.

It will be appreciated that alternative classifiers may be used by the classification module, such as one or more neural networks. In embodiments where the classifier is a neural network, the neural network may be a fully connected neural network, a convolutional neural network, or the like.

While FIG. 1 has been described in the context of EEG signals, it will be appreciated that the method is more generally applicable to other types of signal classification. The input signals 102 may be any type of signal data to be classified.

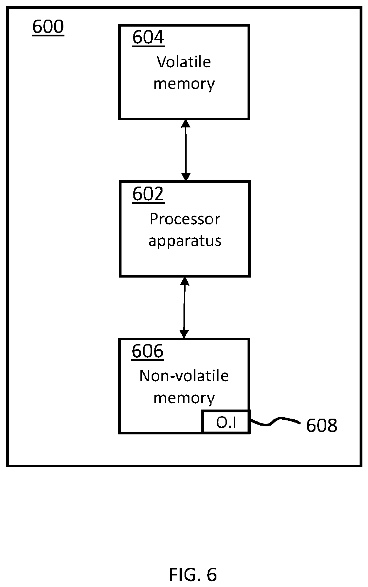

FIG. 3 shows a schematic overview of a method 300 for training a model for classifying/decoding EEG signals, such as the model described above in relation to FIG. 1. The method may be performed by one or more computing systems/apparatus, such as the system described in relation to FIG. 6.

The training method 300 uses a set of labelled training data 324 comprising a plurality of training examples, each of which comprises a plurality of channels of EEG signals and a corresponding ground truth classification for the plurality of channels EEG signals (in the example shown, the classifications are just A and B for convenience). Examples of such datasets include, for example, the SEED dataset (e.g. for emotional recognition)

A batch of training data 322 (or mini-batch) comprising one or more training examples is selected from the training dataset 324. The batch size may, for example, lie in the range [64, 512], for example 256.

Each the plurality of channels of EEG signals 302 of each training example is input into a classification model 328, which generated a candidate classification 330, c, of the plurality of channels EEG signals 302 for that training example.

The classification model 328 comprises a filtering module 304. The filtering module comprises plurality of parametrised filters 306, which are configured to generate a plurality of channels of filtered EEG signals 308 from the plurality of EEG signals of a training example. A differentiable feature module 310 generates plurality of feature maps 314 from the plurality of channels of filtered EEG signals 308 using a differentiable function/mapping 312. The feature maps 314 are input into a parametrised classification module 316, which uses them to determine the candidate classification 330 of the input EEG training example 302. Each of these components operates, for example, as described above in relation to FIG. 1.

The candidate classifications 330 are compared to the ground truth classifications for the corresponding training examples 302 from which they were generated. Based on the comparison, updates to the values of the parameters of the plurality of filters and the classification model are determined.

The comparison may be performed using an objective function, L, 326. The objective function 326 may be based on a difference between candidate classifications 330 and the corresponding ground truth classifications. The objective function may comprise a classification loss. For example, an L2 loss (i.e. a mean squared error) between the candidate classification 330 and the ground truth classification may be used. Other classification losses may alternatively be used, such as a cross entropy loss.

In some embodiments, a lasso regularisation may additionally be included in the objective function. The lasso regularisation may add an L1 norm of the classifier weights, $w_k$, to the objective function with a scaling factor of $\gamma$. $\gamma$ may for example take values of the order $10^{-3}$-$10^{-2}$, e.g. 2e-3, 3e-3 or 3e-2.

An example of such an objective function 326 is given by:

$$\mathcal{L} = \frac{1}{N} \sum_i (x_i - t_i)^2 + \gamma \sum_k |w_k|$$

where x is the candidate classifications, t is the ground truth classifications, i labels the training examples in a batch of $N$ training examples, and $w_k$ are the classification weights.

Updates may, in some embodiments, be determined using batch/mini-batch gradient decent applied to the objective function 326, e.g. mini-batch stochastic gradient descent (SGD) with Nesterov momentum. A heavy momentum may be used on the filter parameters and/or the classifier parameters (e.g. >0.85, such as 0.99 on the filters and 0.9 on the classifier).

Standardization may be performed via batch normalization, which keeps a running mean and variance over mini-batches to approximate dataset statistics. Non-affine batch normalisation may be used, meaning that the target mean and standard deviation are not trainable.

In some embodiments, the learning rate may decay during training. For example, cosine decay to zero on the learning rate may be applied. This ensures that the magnitude of model updates is reduced over time, which can allow batch normalization to capture final statistics of the discovered features.

In embodiments using generalised Gaussian filters, the filters may be initialised with the shape parameter, $\beta$, equal to two. The filters may be initialised at the same centre frequency and have a wide bandwidth (e.g. centred at 23Hz with a bandwidth of 44 Hz). This provides a sufficiently non-zero derivative for all relevant frequencies. During training, the centre frequency, bandwidth and shape parameter may all be updated. A minimum value of the shape parameter of two may be set to prevent the generalised Gaussian moving towards a Laplacian, which can cause problems in training due to its non-continuous derivative. A maximum value of the shape parameter may also be set, for example three.

The shape parameter may be linearly rescaled to accelerate training. For example, the shape parameter may be rescaled as $\beta_{rescale}=8\beta-14$.

The training is iterated with new batches 322 of training examples until a threshold condition is satisfied. The threshold condition may be a threshold number of training epochs. For example, the threshold number of training epochs may be in the range [200, 8000], such as between 300 and 5000, e.g. 300, 1200 or 5000. Alternatively or additionally, the threshold condition may be a threshold performance on a validation dataset. The performance of the model on the validation dataset may be measured using a performance metric, such as the unweighted average recall (UAR), reporting the mean and standard deviation across folds. The UAR for classes $c \in C$, $|C| = N$ is given by

$$UAR = \frac{1}{N} \sum_{c} \frac{true\_positives_c}{true\_positives_c + false\_negatives_c}.$$

The filters may be trained independently for each EEG electrode. Alternatively, when using PLV connectivity, each filter is shared across all electrodes to limit the model to within-frequency band connectivity. Whenever multiple filters per electrode are used (and, hence, multiple feature maps are derived from the same multichannel signal, as shown in FIG. 1), the feature module handles each feature map separately. Interelectrode connections may be considered solely within individual maps. This results in multiple independent feature maps of connectivity, which are then concatenated into a single feature vector for classification.

While FIG. 3 has been described in the context of EEG signals, it will be appreciated that the method is more generally applicable to other types of signal classification. The EEG signals used in the training may be replaced with any type of signal data to be classified in order to train the classifier for that signal classification task.

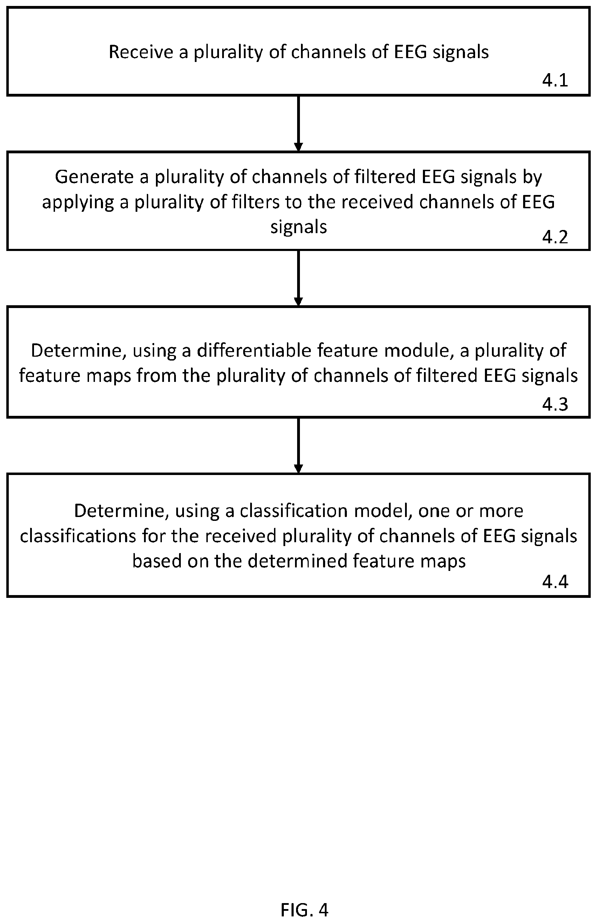

FIG. 4 shows a flow diagram of an example method of classifying/decoding EEG signals. The method may be performed by one or more computing systems, such as the system described below in relation to FIG. 6. The method may correspond to the method described above in relation to FIG. 1.

At operation 4.1, a plurality of channels of EEG signals are received. The plurality of channels of EEG signals may correspond to EEG signals of a human brain measured using a plurality of probes. Each channel of the EEG signals may correspond to measurements taken from a corresponding electrode/probe attached to a human. The signals may be converted into the frequency domain.

At operation 4.2, a plurality of channels of filtered EEG signals are generated by applying a plurality of filters to the received channels of EEG signals. The plurality of filters comprises a plurality of learned parameterised bandpass filters. The parameters of the filters may be learned using a training method, such as the methods described in relation to FIG.s 3 and 5, prior to the use of the filters in the classification method.

The plurality of filters may comprise a plurality of generalised Gaussian filters, such as those described above in relation to Fig. 1.

One or more of the parameters of a filters may control a phase response of the filter. The phase response may be freely controlled. Examples of such phase responses include zero-phase and linear-phase responses, as well as non-linear phase responses.

At operation 4.3, a plurality of feature maps are generated from the plurality of channels of filtered EEG signals using a differentiable feature module.

Each of a plurality of feature maps in the plurality of feature maps may be associated with a respective plurality of channels of filtered EEG channels. These filtered EEG channels correspond to the received plurality of channels of EEG signals. Each of the respective plurality of channels of filtered EEG channels is determined by applying a respective plurality of filters to the plurality of channels of EEG signals. In other words, each feature map corresponds to a different filtered version of the received plurality of EEG channels.

The one or more feature maps comprises one or more measures of functional connectivity between EEG signals in the plurality of filtered EEG signals. For example, determining the one or more feature maps may comprise determining a connectivity matrix between two or more of the filtered EEG signals. A feature vector is then extracted from the connectivity matrix, which may correspond to an upper triangular (or lower triangular) of the connectivity matrix. This extracted feature vector is input into the classification model. Where multiple feature vectors are extracted, they may be standardised and concatenated before input into the classification model.

The one or more measures of functional connectivity between filtered EEG signals may comprise one or more of: a correlation function between two of the filtered EEG signals; a phase locking value; amplitude envelope correlations; and/or signal envelope correlations.

Alternatively or additionally, the one or more feature maps may comprise one or more of: a measure of magnitude of each of the filtered EEG signals; a signal power; a signal variance; and/or a signal entropy. The measure of magnitude of each of the filtered EEG signals comprises a sum of the magnitude of the filtered EEG signal over frequency bins of the filtered EEG signal, as described above in relation to FIG. 1.

At operation 4.4, a classification model is applied to the determined feature maps to determine one or more classifications for the received plurality of channels of EEG signals.

The one or more classifications for the plurality of channels of EEG signals may comprise one or more of: a classification of a resting or active state; a classification of a dynamic state triggered by/underlying the physical or imaginary movement of extremities; a classification of a dynamic state triggered by/underlying a conscious or non-conscious cognitive process related to attention tasks, perception tasks, planning tasks, memory tasks, language tasks, arithmetic tasks, reading tasks, control interface tasks, and specialized tasks like flight or driving, either in a simulator or in a real vehicle action; a classification of an affective state; a classification of an anomaly; a classification of a control intention for an external device; and/or a classification of clinical states.

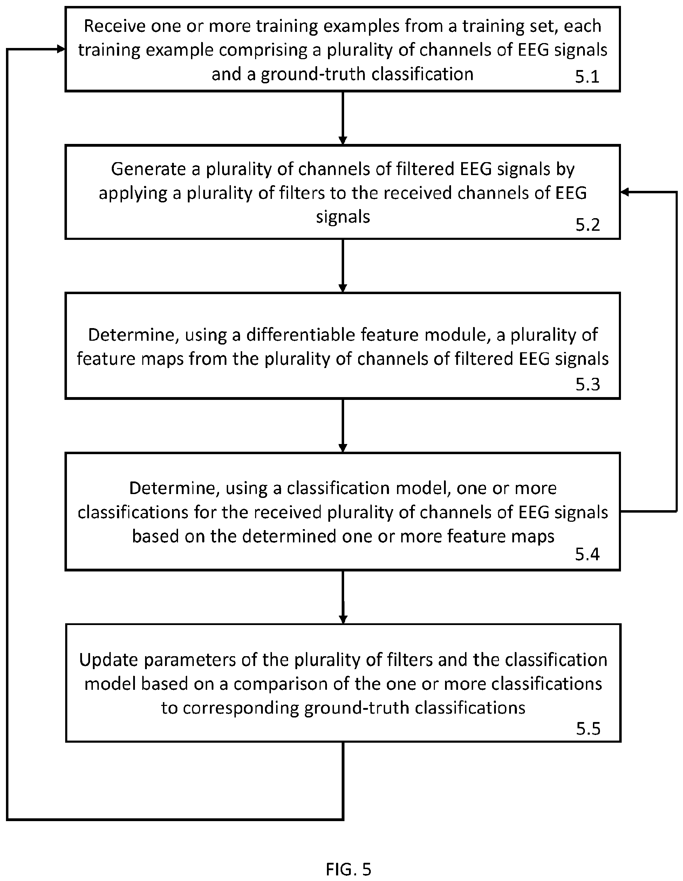

FIG. 5 shows a flow diagram of an example method of training a model for classifying EEG signals.

At operation 5.1, one or more training examples from a training set are received. The one or more training examples may form a training batch/mini-batch. Each training example comprising a plurality of channels of EEG signals and a corresponding ground-truth classification.

At operation 5.2, a plurality of channels of filtered EEG signals are generated by applying a plurality of filters to the plurality of EEG signals of a training example. The plurality of filters comprises a plurality of parametrised bandpass filters, as described above in relation to FIG.s 1-4.

At operation 5.3, a differentiable feature module is used to determine a plurality of feature maps from the plurality of channels of filtered EEG signals. The differentiable feature module applies one or more fixed functions to the filtered EEG signals to determine the feature map, as described above in relation to FIG.s 1-4.

At operation 5.4, a classification model is used to determine one or more (candidate) classifications for the plurality of channels of EEG signals of said training example based on the determined feature maps. The classification model is a parametrised classification model, as described above in relation to FIG.s 1-4.

Operations 5.2 to 5.4 are iterated over the one or more training examples until a candidate classification for each of the training examples has been determined.

At operation 5.5, parameters of the plurality of filters and the classification model are updated based on a comparison of the one or more classifications to corresponding ground-truth classifications. The comparison may be made using an objective function. The updates may be determined using backpropagation of gradients.

The objective function may comprise a comparison term comprising a norm of a difference between the candidate classifications and corresponding ground-truth classifications. The norm may be an L2 norm, and L1 norm or the like. Other classification losses may alternatively be used as an objective function. The objective function further comprises a regularisation term comprising a sum of norms of weights (i.e. parameters) of the classification model. Examples of objective functions are described in more detail above with respect to FIG. 3.

Operations 5.1 to 5.5 are iterated over the training dataset until a threshold condition is satisfied. The threshold condition may be a threshold number of training epochs and/or a threshold performance on a validation dataset.

While the forgoing systems and methods have been described in the context of filtering and classifying EEG signals, it will be appreciated by the skilled person that the systems and methods described herein can be applied to the classification of many other types of input signal. In the field of brain activity, other brain activity signals may alternatively or additionally be used as input to the method, such as magnetoencephalography (MEG) signals.

More generally, the classification methods and training methods described herein may be applied to signal data outside of the field of brain activity. Examples of such applications include, for example: audio classification, where the input to the classifier is a plurality of channels of audio data and the output is one more classifications of the audio data; sensor classification, where the input to the classifier is a plurality of channels of sensor/telemetry data and the output is one more classifications of the sensor/telemetry data (e.g. whether an anomaly is present); and physiological data, such as ECG signals, where the input to the classifier is a plurality of channels of physiological data and the output is one more classifications of the physiological data (e.g. whether a health condition is present). Many other examples will be familiar to the person skilled in the art. In particular, the use of generalised Gaussians as filters in these applications can improve the training and accuracy of the classifier, and allow the classifier to be trained end-to-end while identifying interpretable features.

FIG. 6 shows a schematic example of a system/apparatus for performing any of the methods described herein. The system/apparatus shown is an example of a computing device. It will be appreciated by the skilled person that other types of computing devices/systems may alternatively be used to implement the methods described herein, such as a distributed computing system.

The apparatus (or system) 600 comprises one or more processors 602. The one or more processors control operation of other components of the system/apparatus 600. The one or more processors 602 may, for example, comprise a general-purpose processor. The one or more processors 602 may be a single core device or a multiple core device. The one or more processors 602 may comprise a Central Processing Unit (CPU) or a graphical processing unit (GPU). Alternatively, the one or more processors 1202 may comprise specialised processing hardware, for instance a RISC processor or programmable hardware with embedded firmware. Multiple processors may be included.

The system/apparatus comprises a working or volatile memory 604. The one or more processors may access the volatile memory 604 in order to process data and may control the storage of data in memory. The volatile memory 604 may comprise RAM of any type, for example Static RAM (SRAM), Dynamic RAM (DRAM), or it may comprise Flash memory, such as an SD-Card.

The system/apparatus comprises a non-volatile memory 606. The non-volatile memory 606 stores a set of operation instructions 608 for controlling the operation of the processors 602 in the form of computer readable instructions. The non-volatile memory 606 may be a memory of any kind such as a Read Only Memory (ROM), a Flash memory or a magnetic drive memory.

The one or more processors 602 are configured to execute operating instructions 608 to cause the system/-apparatus to perform any of the methods described herein. The operating instructions 608 may comprise code (i.e. drivers) relating to the hardware components of the system/apparatus 600, as well as code relating to the basic operation of the system/apparatus 600. Generally speaking, the one or more processors 602 execute one or more instructions of the operating instructions 608, which are stored permanently or semi-permanently in the non-volatile memory 606, using the volatile memory 604 to store temporarily data generated during execution of said operating instructions 608.

Implementations of the methods described herein may be realised as in digital electronic circuitry, integrated circuitry, specially designed ASICs (application specific integrated circuits), computer hardware, firmware, software, and/or combinations thereof. These may include computer program products (such as software stored on e.g. magnetic discs, optical disks, memory, Programmable Logic Devices) comprising computer readable instructions that, when executed by a computer, such as that described in relation to Figure 6, cause the computer to perform one or more of the methods described herein.

Any system feature as described herein may also be provided as a method feature, and vice versa. As used herein, means plus function features may be expressed alternatively in terms of their corresponding structure. In particular, method aspects may be applied to system aspects, and vice versa.

Although several embodiments have been shown and described, it would be appreciated that the invention is not limited by these embodiments but it is defined by the appended claims.

**Claims**

1. A computer implemented method of classifying brain activity signals, the method comprising:

   receiving a plurality of channels of brain activity signals (102);
   generating a plurality of channels of filtered brain activity signals (108) by applying a plurality of filters (106) to the received channels of brain activity signals, wherein the plurality of filters comprises a plurality of trained parameterised bandpass filters;
   determining, using an untrained differentiable feature module (110), a plurality of feature maps (114) from the plurality of channels of filtered brain activity signals; and
   determining, using a classification model (116), one or more classifications for the received plurality of channels of brain activity signals based on the determined feature maps.

2. A computer implemented method of training a model for classifying brain activity signals, the method comprising:

   for each of one or more of training examples (322) from a training set (324), each training example comprising a plurality of channels of brain activity signals (302) and a ground-truth classification:

   generating a plurality of channels of filtered brain activity signals (308) by applying a plurality of filters (306) to the plurality of brain activity signals of said training example, wherein the plurality of filters comprises a plurality of parametrised bandpass filters;
   determining, using an untrained differentiable feature module (310), a plurality of feature maps (314) from the plurality of channels of filtered brain activity signals, wherein the differentiable feature module applies one or more fixed functions (312) to the filtered brain activity signals to determine the feature maps; and determining, using a classification model (316), one or more candidate classifications (330) for the plurality of channels of brain activity signals of said training example based on the determined feature maps, and

updating parameters of the plurality of filters and the classification model based on a comparison of the one or more candidate classifications to corresponding ground-truth classifications, wherein the comparison is made using an objective function (326) and wherein the updates are determined using backpropagation of gradients.

3. The method of claim 2, wherein the objective function (326) comprises a comparison term comprising a norm of a difference between the classifications and corresponding ground-truth classifications.

4. The method of claim 3, wherein the objective function (326) further comprises a regularisation term comprising a sum of norms of weights of the classification model.

5. The method of any preceding claim, wherein each of a plurality of feature maps in the plurality of feature maps (114) is associated with a respective plurality of channels of filtered EEG channels corresponding to the plurality of channels of brain activity signals, and wherein each of the respective plurality of channels of filtered brain activity channels is determined by applying a respective plurality of filters to the plurality of channels of EEG signals.

6. The method of any preceding claim, wherein the one or more feature maps (114) comprises one or more measures of functional connectivity between brain activity signals in the plurality of filtered brain activity signals (102).

7. The method of claim 6, wherein determining the one or more feature maps (114) comprises:

determining a connectivity matrix between two or more of the filtered brain activity signals; and
extracting a feature vector from the connectivity matrix, wherein the feature vector corresponds to an upper triangular or lower triangular of the connectivity matrix, and
wherein determining, using the classification model (116), the one or more classifications for the plurality of channels of brain activity signals the comprises inputting the extracted feature vector into the classification model.

8. The method of any of claims 6 or 7, wherein the one or more measures of functional connectivity between filtered brain activity signals comprises one or more of: a correlation function between two of the filtered brain activity signals; a phase locking value; amplitude envelope correlations; and/or signal envelope correlations.

9. The method of any preceding claim, wherein the one or more feature maps (114) comprises one or more of: a measure of magnitude of each of the filtered brain activity signals; a signal power; a signal variance; and/or a signal entropy.

10. The method of claim 9, wherein the measure of magnitude of each of the filtered brain activity signals comprises a sum of the magnitude of the filtered brain activity signal over frequency bins of the filtered brain activity signal.

11. The method of any preceding claim, wherein the one or more classifications for the plurality of channels of brain activity signals comprises: a classification of a resting or active state; a classification of a dynamic state triggered by/underlying the physical or imaginary movement of extremities; a classification of a dynamic state triggered by/underlying a conscious or non-conscious cognitive process related to attention tasks, perception tasks, planning tasks, memory tasks, language tasks, arithmetic tasks, reading tasks, control interface tasks, and specialized tasks like flight or driving, either in a simulator or in a real vehicle action; a classification of an affective state; a classification of an anomaly; a classification of a control intention for an external device; and/or a classification of clinical states.

12. The method of any preceding claim, wherein the plurality of filters (106) comprises a plurality of generalised Gaussian filters.

13. The method of any preceding claim, wherein the brain activity signals (102) are EEG signals.

14. A computer program product comprising computer-readable code that, when executed by a computing system, causes the computing system to perform a method according to any preceding claim.

15. A system comprising one or more processors (602) and a memory (606), the memory storing computer readable instructions (608) that, when executed by the one or more processors, causes the system to perform a method according to any of claims 1-13.

**Patentansprüche**

1. Computerimplementiertes Verfahren zum Klassifizieren von Gehirnaktivitätssignalen, wobei das Verfahren Folgendes umfasst:

   Empfangen einer Vielzahl von Kanälen von Gehirnaktivitätssignalen (102);
   Erzeugen einer Vielzahl von Kanälen gefilterter Gehirnaktivitätssignale (108) durch Anwenden einer Vielzahl von Filtern (106) auf die empfangenen Kanäle von Gehirnaktivitätssignalen, wobei die Vielzahl von Filtern eine Vielzahl von trainierten parametrisierten Bandpassfiltern umfasst;
   Bestimmen einer Vielzahl von Merkmalskarten (114) aus der Vielzahl von Kanälen gefilterter Gehirnaktivitätssignale unter Verwendung eines untrainierten differenzierbaren Merkmalsmoduls (110); und
   Bestimmen, unter Verwendung eines Klassifizierungsmodells (116), einer oder mehrerer Klassifizierungen für die empfangene Vielzahl von Kanälen von Gehirnaktivitätssignalen basierend auf den bestimmten Merkmalskarten.

2. Computerimplementiertes Verfahren zum Trainieren eines Modells zum Klassifizieren von Gehirnaktivitätssignalen, wobei das Verfahren Folgendes umfasst:
   für jedes von einem oder mehreren von Trainingsbeispielen (322) aus einem Trainingssatz (324), wobei jedes Trainingsbeispiel eine Vielzahl von Kanälen von Gehirnaktivitätssignalen (302) und eine Ground-Truth-Klassifizierung umfasst:

   Erzeugen einer Vielzahl von Kanälen gefilterter Gehirnaktivitätssignale (308) durch Anwenden einer Vielzahl von Filtern (306) auf die Vielzahl von Gehirnaktivitätssignalen des Trainingsbeispiels, wobei die Vielzahl von Filtern eine Vielzahl von parametrisierten Bandpassfiltern umfasst;
   Bestimmen einer Vielzahl von Merkmalskarten (314) aus der Vielzahl von Kanälen gefilterter Gehirnaktivitätssignale unter Verwendung eines untrainierten differenzierbaren Merkmalsmoduls (310), wobei das differenzierbare Merkmalsmodul eine oder mehrere feste Funktionen (312) auf die gefilterten Gehirnaktivitätssignale anwendet, um die Merkmalskarten zu bestimmen; und
   Bestimmen, unter Verwendung eines Klassifizierungsmodells (316), einer oder mehrerer Kandidatenklassifizierungen (330) für die Vielzahl von Kanälen von Gehirnaktivitätssignalen des Trainingsbeispiels basierend auf den bestimmten Merkmalskarten und
   Aktualisieren von Parametern der Vielzahl von Filtern und des Klassifizierungsmodells basierend auf einem Vergleich der einen oder der mehreren Kandidatenklassifizierungen mit entsprechenden Ground-Truth-Klassifizierungen, wobei der Vergleich unter Verwendung einer Zielfunktion (326) durchgeführt wird und wobei die Aktualisierungen unter Verwendung von Rückwärtspropagation von Gradienten bestimmt werden.

3. Verfahren nach Anspruch 2, wobei die Zielfunktion (326) einen Vergleichsterm umfasst, der eine Norm einer Differenz zwischen den Klassifizierung und entsprechenden Ground-Truth-Klassifizierungen umfasst.

4. Verfahren nach Anspruch 3, wobei die Zielfunktion (326) ferner einen Regularisierungsterm umfasst, der eine Summe von Normen von Gewichten des Klassifizierungsmodells umfasst.

5. Verfahren nach einem vorhergehenden Anspruch, wobei jede einer Vielzahl von Merkmalskarten in der Vielzahl von Merkmalskarten (114) einer jeweiligen Vielzahl von Kanälen gefilterter EEG-Kanäle zugeordnet ist, die der Vielzahl von Kanälen von Gehirnaktivitätssignalen entsprechen, und wobei jeder der jeweiligen Vielzahl von Kanälen gefilterter Gehirnaktivitätskanäle durch Anwenden einer jeweiligen Vielzahl von Filtern auf die Vielzahl von Kanälen von EEG-Signalen bestimmt wird.

6. Verfahren nach einem vorhergehenden Anspruch, wobei die eine oder mehreren Merkmalskarten (114) eine oder mehrere Messungen einer funktionellen Konnektivität zwischen Gehirnaktivitätssignalen in der Vielzahl von gefilterten Gehirnaktivitätssignalen (102) umfassen.

7. Verfahren nach Anspruch 6, wobei das Bestimmen der einen oder mehreren Merkmalskarten (114) Folgendes umfasst:

Bestimmen einer Konnektivitätsmatrix zwischen zwei oder mehr der gefilterten Gehirnaktivitätssignale; und Extrahieren eines Merkmalsvektors aus der Konnektivitätsmatrix, wobei der Merkmalsvektor einem oberen Dreieck oder unteren Dreieck der Konnektivitätsmatrix entspricht, und

wobei das Bestimmen, unter Verwendung des Klassifizierungsmodells (116), der einen oder mehreren Klassifizierungen für die Vielzahl von Kanälen von Gehirnaktivitätssignalen Eingeben des extrahierten Merkmalsvektors in das Klassifizierungsmodell umfasst.

8. Verfahren nach einem der Ansprüche 6 oder 7, wobei das eine oder die mehreren Messungen einer funktionellen Konnektivität zwischen gefilterten Gehirnaktivitätssignalen eines oder mehrere von Folgenden umfassen: eine Korrelationsfunktion zwischen zwei der gefilterten Gehirnaktivitätssignale; einen Phasenverriegelungswert; Amplitudenhüllkurvenkorrelationen; und/oder Signalhüllkurvenkorrelationen.

9. Verfahren nach einem vorhergehenden Anspruch, wobei die eine oder mehreren Merkmalskarten (114) eines oder mehrere von Folgenden umfassen: eine Messung einer Größe jedes der gefilterten Gehirnaktivitätssignale; eine Signalleistung; eine Signalvarianz; und/oder eine Signalentropie.

10. Verfahren nach Anspruch 9, wobei die Messung einer Größe jedes der gefilterten Gehirnaktivitätssignale eine Summe der Messung der Größe des gefilterten Gehirnaktivitätssignals über Frequenz-Bins des gefilterten Gehirnaktivitätssignals umfasst.

11. Verfahren nach einem vorhergehenden Anspruch, wobei die eine oder mehreren Klassifizierungen für die Vielzahl von Kanälen von Gehirnaktivitätssignalen Folgendes umfassen: eine Klassifizierung eines Ruhe- oder aktiven Zustands; eine Klassifizierung eines dynamischen Zustands, der durch die physische oder imaginäre Bewegung von Extremitäten ausgelöst wird/dieser unterliegt; eine Klassifizierung eines dynamischen Zustands, der durch einen bewussten oder unbewussten kognitiven Prozess ausgelöst wird/diesem unterliegt, der sich auf Aufmerksamkeitsaufgaben, Wahrnehmungsaufgaben, Planungsaufgaben, Gedächtnisaufgaben, Sprachaufgaben, arithmetische Aufgaben, Leseaufgaben, Steuerschnittstellenaufgaben und spezialisierte Aufgaben wie Flug oder Fahren bezieht, entweder in einem Simulator oder in einer realen Fahrzeugaktion; eine Klassifizierung eines affektiven Zustands; eine Klassifizierung einer Anomalie; eine Klassifizierung einer Steuerabsicht für eine externe Vorrichtung; und/oder eine Klassifizierung klinischer Zustände.

12. Verfahren nach einem vorhergehenden Anspruch, wobei die Vielzahl von Filtern (106) eine Vielzahl von generalisierten Gauß-Filtern umfasst.

13. Verfahren nach einem vorhergehenden Anspruch, wobei die Gehirnaktivitätssignale (102) EEG-Signale sind.

14. Computerprogrammprodukt, umfassend einen computerlesbaren Code, der, wenn er durch ein Rechensystem ausgeführt wird, das Rechensystem veranlasst, ein Verfahren nach einem vorhergehenden Anspruch durchzuführen.

15. System, umfassend einen oder mehrere Prozessoren (602) und einen Speicher (606), wobei der Speicher computerlesbare Anweisungen (608) speichert, die, wenn sie durch den einen oder die mehreren Prozessoren ausgeführt werden, das System dazu veranlassen, ein Verfahren nach einem der Ansprüche 1-13 durchzuführen.

## Revendications

1. Procédé mis en œuvre par ordinateur de classification de signaux d'activité cérébrale, le procédé comprenant :

la réception d'une pluralité de canaux de signaux d'activité cérébrale (102) ;
la génération d'une pluralité de canaux de signaux d'activité cérébrale filtrés (108) en appliquant une pluralité de filtres (106) aux canaux reçus de signaux d'activité cérébrale, dans lequel la pluralité de filtres comprennent une pluralité de filtres passe-bande paramétrés entraînés ;
la détermination, à l'aide d'un module de caractéristiques différentiables non entraîné (110), d'une pluralité de cartes de caractéristiques (114) à partir de la pluralité de canaux de signaux d'activité cérébrale filtrés ; et
la détermination, à l'aide d'un modèle de classification (116), d'une ou plusieurs classifications pour la pluralité reçue de canaux de signaux d'activité cérébrale sur la base des cartes de caractéristiques déterminées.

**2.** Procédé mis en œuvre par ordinateur d'entraînement d'un modèle de classification de signaux d'activité cérébrale, le procédé comprenant :
pour chacun d'un ou plusieurs exemples d'entraînement (322) issus d'un ensemble d'entraînement (324), chaque exemple d'entraînement comprenant une pluralité de canaux de signaux d'activité cérébrale (302) et une classification de référence :

la génération d'une pluralité de canaux de signaux d'activité cérébrale filtrés (308) en appliquant une pluralité de filtres (306) à la pluralité de signaux d'activité cérébrale dudit exemple d'entraînement, dans lequel la pluralité de filtres comprennent une pluralité de filtres passe-bande paramétrés ;
la détermination, à l'aide d'un module de caractéristiques différentiables non entraîné (310), d'une pluralité de cartes de caractéristiques (314) issues de la pluralité de canaux de signaux d'activité cérébrale filtrés, dans lequel le module de caractéristiques différentiables applique une ou plusieurs fonctions fixes (312) aux signaux d'activité cérébrale filtrés pour déterminer les cartes de caractéristiques ; et
la détermination, à l'aide d'un modèle de classification (316), d'une ou plusieurs classifications candidates (330) pour la pluralité de canaux de signaux d'activité cérébrale dudit exemple d'entraînement sur la base des cartes de caractéristiques déterminées, et
la mise à jour des paramètres de la pluralité de filtres et du modèle de classification sur la base d'une comparaison de l'une ou plusieurs classifications candidates aux classifications de référence correspondantes, dans lequel la comparaison est effectuée à l'aide d'une fonction objective (326) et dans lequel les mises à jour sont déterminées à l'aide d'une rétropropagation de gradients.

**3.** Procédé selon la revendication 2, dans lequel la fonction objective (326) comprend un terme de comparaison comprenant une norme d'une différence entre les classifications et les classifications de référence correspondantes.

**4.** Procédé selon la revendication 3, dans lequel la fonction objective (326) comprend en outre un terme de régularisation comprenant une somme de normes de poids du modèle de classification.

**5.** Procédé selon l'une quelconque des revendications précédentes, dans lequel chacune d'une pluralité de cartes de caractéristiques dans la pluralité de cartes de caractéristiques (114) est associée à une pluralité respective de canaux de canaux EEG filtrés correspondant à la pluralité de canaux de signaux d'activité cérébrale, et dans lequel chacun de la pluralité respective de canaux d'activité cérébrale filtrés est déterminé en appliquant une pluralité respective de filtres à la pluralité de canaux de signaux EEG.

**6.** Procédé selon l'une quelconque des revendications précédentes, dans lequel l'une ou plusieurs cartes de caractéristiques (114) comprennent une ou plusieurs mesures de connectivité fonctionnelle entre les signaux d'activité cérébrale dans la pluralité de signaux d'activité cérébrale filtrés (102).

**7.** Procédé selon la revendication 6, dans lequel la détermination de l'une ou plusieurs cartes de caractéristiques (114) comprend :

la détermination d'une matrice de connectivité entre deux signaux d'activité cérébrale filtrés ou plus ; et
l'extraction d'un vecteur de caractéristique de la matrice de connectivité, dans lequel le vecteur de caractéristique correspond à une triangulaire supérieure ou une triangulaire inférieure de la matrice de connectivité, et dans lequel la détermination, à l'aide du modèle de classification (116), de l'une ou plusieurs classifications pour la pluralité de canaux de signaux d'activité cérébrale le comprend l'entrée du vecteur de caractéristiques extrait dans le modèle de classification.

**8.** Procédé selon l'une quelconque des revendications 6 ou 7, dans lequel l'une ou plusieurs mesures de connectivité fonctionnelle entre les signaux d'activité cérébrale filtrés comprennent une ou plusieurs parmi : une fonction de corrélation entre deux des signaux d'activité cérébrale filtrés ; une valeur de verrouillage de phase ; des corrélations d'enveloppe d'amplitude ; et/ou des corrélations d'enveloppe de signal.

**9.** Procédé selon l'une quelconque des revendications précédentes, dans lequel l'une ou plusieurs cartes de caractéristiques (114) comprennent une ou plusieurs parmi : une mesure de l'amplitude de chacun des signaux d'activité cérébrale filtrés ; une puissance de signal ; une variance de signal ; et/ou une entropie de signal.

**10.** Procédé selon la revendication 9, dans lequel la mesure de l'amplitude de chacun des signaux d'activité cérébrale filtrés comprend une somme de l'amplitude du signal d'activité cérébrale filtré sur des tranches de fréquence du signal d'activité cérébrale filtré.

**11.** Procédé selon l'une quelconque des revendications précédentes, dans lequel l'une ou plusieurs classifications pour la pluralité de canaux de signaux d'activité cérébrale comprennent : une classification d'un état de repos ou actif ; une classification d'un état dynamique déclenché par/sous-jacent au mouvement physique ou imaginaire des extrémités ; une classification d'un état dynamique déclenché par/sous-jacent à un processus cognitif conscient ou non conscient lié aux tâches d'attention, aux tâches de perception, aux tâches de planification, aux tâches de mémoire, aux tâches linguistiques, aux tâches arithmétiques, aux tâches de lecture, aux tâches d'interface de contrôle et aux tâches spécialisées comme le vol ou la conduite, soit dans un simulateur ou en action réelle en véhicule ; une classification d'un état affectif ; une classification d'une anomalie ; une classification d'une intention de contrôle pour un dispositif externe ; et/ou une classification d'états cliniques.

**12.** Procédé selon l'une quelconque des revendications précédentes, dans lequel la pluralité de filtres (106) comprennent une pluralité de filtres gaussiens généralisés.

**13.** Procédé selon l'une quelconque des revendications précédentes, dans lequel les signaux d'activité cérébrale (102) sont des signaux EEG.

**14.** Produit programme informatique comprenant un code lisible par ordinateur qui, lorsqu'il est exécuté par un système informatique, amène le système informatique à exécuter un procédé selon l'une quelconque des revendications précédentes.

**15.** Système comprenant un ou plusieurs processeurs (602) et une mémoire (606), la mémoire stockant des instructions lisibles par ordinateur (608) qui, lorsqu'elles sont exécutées par l'un ou plusieurs processeurs, amènent le système à mettre en œuvre un procédé selon l'une quelconque des revendications 1 à 13.

FIG. 1

Ideal sinc

Frequency [Hz]

FIG. 2a

sinc k=129

Frequency [Hz]

FIG. 2b

Gen. Gaussian β=2

Frequency [Hz]

FIG. 2c

Gen. Gaussian β=10

Frequency [Hz]

FIG. 2d

FIG. 3

Receive a plurality of channels of EEG signals

4.1

Generate a plurality of channels of filtered EEG signals by applying a plurality of filters to the received channels of EEG signals

4.2

Determine, using a differentiable feature module, a plurality of feature maps from the plurality of channels of filtered EEG signals

4.3

Determine, using a classification model, one or more classifications for the received plurality of channels of EEG signals based on the determined feature maps

4.4

FIG. 4

Receive one or more training examples from a training set, each training example comprising a plurality of channels of EEG signals and a ground-truth classification 5.1

Generate a plurality of channels of filtered EEG signals by applying a plurality of filters to the received channels of EEG signals 5.2

Determine, using a differentiable feature module, a plurality of feature maps from the plurality of channels of filtered EEG signals 5.3

Determine, using a classification model, one or more classifications for the received plurality of channels of EEG signals based on the determined one or more feature maps 5.4

Update parameters of the plurality of filters and the classification model based on a comparison of the one or more classifications to corresponding ground-truth classifications 5.5

FIG. 5

FIG. 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **D. BORRA et al.** *Neural Netw*, September 2020, vol. 129, 55-74

- **R. T. SCHIRRMEISTER et al.** *Hum. Brain Mapp.*, vol. 38, 5391-5420